# EUROPEAN PATENT APPLICATION

(11) **EP 3 505 164 A1**
(43) Date of publication of application: **03.07.2019**
(21) Application number: 17020598.3
(22) Date of filing: 31.12.2017
(51) Int. Cl.: A61K 9/46, A61K 9/20, A61K 31/381, A61P 11/12

(54) **AN EFFERVESCENT COMPOSITION COMPRISING ERDOSTEINE**

(71) Applicant: Abdi Ibrahim Ilac Sanayi ve Ticaret A.S., 34500 Esenyurt/Istanbul (TR)
(72) Inventor: Koc, Erhan, Esenyurt - Istanbul (TR); Aktas, Tolga, Esenyurt - Istanbul (TR)

(57) **Abstract**

Invention relates to effervescent pharmaceutical composition comprising active pharmaceutical ingredient Erdosteine and / or pharmaceutically acceptable salts, esters, polymorphs, solvates, enantiomers and / or derivatives thereof, at least one effervescent acid-base couple and at least one pharmaceutically acceptable excipient.

## Description

### Technical Field

Invention relates to effervescent pharmaceutical composition comprising active pharmaceutical ingredient Erdosteine and / or pharmaceutically acceptable salts, esters, polymorphs, solvates, enantiomers and / or derivatives thereof, at least one effervescent acid-base couple and at least one pharmaceutically acceptable excipient.

### Background of the Invention

According to IUPAC nomenclature system, Erdosteine is named as 2-[2-oxo-2-[(2-oxothiolan-3-yl)amino]ethyl]sulfanylacetic acid and its chemical formula is shown as in Formula I below.

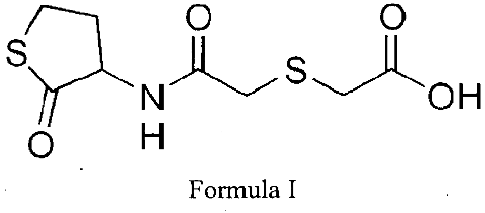

Erdostein is available in capsule form at 150 mg and 300 mg doses and in dry powder form (175 mg / 5 ml) for suspension use in the market. Erdostein, a thiol-derived mucolytic agent, is used in the treatment of chronic obstructive pulmonary disease (COPD), including acute infective exacerbation of chronic bronchitis

Erdostin acts as a mucolytic agent by the release of thiol groups in its structure in metabolism. Activated Erdosteine is effective in lowering the viscosity of mucus and purulent sputum. For this reason, Erdosteine is used as an expectorant in COPD patients. The reduction of the viscosity of said mucus and sputum is due to the opening of disulfide bonds of bronchial mucoproteins. Thus, mucus secretion and viscosity are regulated, expectoration occurs due to the increase mucociliary transport. The active metabolites of Erdostein have activity in the removal of free radicals as well as mucolytic properties. Thus, the protection against the effects of free radicals produced by cigarette smoke is provided. Furthermore, due to the presence of free thiol groups in Erdosteine's active metabolite, Erdostesine has an important antioxidant effect proven both in vitro and in vivo studies.

Although Erdosteine is used in different oral dosage forms up to today in the technical field, it has not been used in the form of effervescent tablets. The most important reason for this is that the solubility values of Erdosteine (especially in water) are so low to be used in effervescent tablets.

Effervescent tablets are administered by dissolving in a liquid (preferably water) rapidly. In this sense, it does not seem possible to produce Erdosteine in the form of an effervescent tablet to reach the desired solubility values without the selection of suitable acid-base components and the optimization of the amount of use. In addition, dissolution of the excipients in the pharmaceutical composition is also important. In some effervescent compositions, due to the absence of the appropriate effervescent acid-base pair, reaction may occur between acid and base that affects the organoleptic quality negatively such as unclear solution formation and solid salt accumulation in the cup bottom.

On the other hand, compounds which contains thiol groups in their content, such as Erdosteine, generally have an unpleasant odor and are frequently removed orally after intake, especially in small children and in the elderly, due to their malodor. This leads to the decreasing of performance provided from the drug containing thiol groups and prolongation of the disease duration.

Another problem in the technical field is the disadvantages of solid oral dosage forms such as tablet, capsules. One of the disadvantages mentioned is the difficulty of these dosage forms. For example, pediatric and geriatric patients groups, patients with mental disorders, people who experiences acute allergic reaction, patients who suffer from nausea experience swallowing difficulties, which leads to reduction the frequency of drug usage and decrease in the yield provided from the drug.

In addition, in the said dosage forms due to the drug's taste / dose is not adjustable by the patient and its bitter or unwanted tastes lead not to use the drug. For all these reasons, the use of these dosage forms generally do not meet the expectation of obtaining benefit from the drug.

Taking into consideration the problems mentioned above in the prior art, there is a need for improvements and additional advantages that solve the problems.

### Brief Description of the Invention

Present invention relates to effervescent pharmaceutical composition comprising Erdosteine, which meets the needs mentioned above, eliminates the disadvantages, and offers additional advantages.

The main objective of the invention is to produce Erdosteine effervescent tablet which is not produced in effervescent form before due to its low solubility properties by selecting suitable acid-base pair and the excipients. Thus, effervescent pharmaceutical composition having high homogenity, high stability, clear appearance and safety dose is obtained.

Another objective of the invention is to obtain pharmaceutical composition that enables faster absorption in comparison to solid oral dosage forms. By dissolving Erdosteine in water, the disadvantages of solid forms such as slow absorption and its effects are eliminated. Thus, it is possible to use lower doses in the treatment process.

Another objective of the invention is to obtain effervescent pharmaceutical composition without the need of using extra sugar due to the sucralose in the composition. Thus, each type of patients including diabetics can use the drug safely. At the same time, effervescent tablet is advantageous in terms of drug delivery and treatment, for patients who are not prone to use orally (such as young children, people who have difficulty in swallowing, mental illness, elderly people), and the taste of the drug is adjustable by the patient.

### Detailed Description of the Invention

In detailed description, preferred embodiments of the invention are disclosed only for the purpose of better understanding of the invention without any limitation.

Invention relates to pharmaceutical composition comprising Erdosteine as active pharmaceutical ingredient, at least one effervescent acid-base couple and at least one pharmaceutically acceptable excipient to be used in respiratory diseases such as chronic obstructive pulmonary disease.

The term of "Erdosteine" refers to Erdosteine active pharmaceutical ingredient, pharmaceutically acceptable salts, esters, solvates, polymorphs , enantiomers, derivatives or mixtures thereof.

The effervescent acid-base couple in the pharmaceutical composition of the invention consists of at least one acid and at least one base.

At least one excipient used in the pharmaceutical composition of the invention is selected from filler, sweetener, lubricant, binder and/or mixtures thereof.

In one embodiment of the invention, acidic component of the effervescent acid-base couple is selected from citric acid, tartaric acid, absorbic acid, fumaric acid, malic acid, adipic acid and/or mixtures thereof and is not limited thereto. Preferably, citric acid derivatives, citric acid anhydride and citric acid monohydride, are used together in the pharmaceutical composition.

In one embodiment of the invention, basic component of the effervescent acid-base couple is selected from sodium bicarbonate, sodium carbonate, potassium bicarbonate, potassium carbonate, potassium carbonate micronised, sesquicarbonate, amorphous calcium carbonate, calcium carbonate and/or mixtures thereof and is not limited thereto. Preferably, sodium carbonate and potassium carbonate are used.

In a preferred embodiment of the invention, basic component of effervescent acid-base couple, potassium carbonate, is used in micronized form wherein mean particle size(d₅₀) of it is between 100 µm and 400 µm, preferably between 250 µm and 300 µm and d₉₀ particle size of it is between 400 µm and 700 µm, preferably between 500 µm and 600 µm.

As used herein, the term "d₅₀ particle size" refers to the average particle size of the particles and refers to average particle diameter by the volume. The term "d₉₀ particle size" refers to particle diameter belong to %90 of the particles by volume. d₅₀ and d₉₀ values are measured by one of the known measurement methods such as laser diffraction by laser diffraction device (eg. Malvern Mastersizer). Measurements can be performed by wet and dry methods. Preferably the dry method is used.

The filler in the pharmaceutical composition fills the pharmaceutical composition, making it practical for a pharmaceutical composition to be produced and available for use by the consumer. The filler in the present invention refers to calcium carbonate, calcium phosphate, dibasic calcium phosphate, tribasic calcium sulfate, calcium carboxymethyl cellulose, cellulose, dextrin derivatives, dextrin, dextrose, fructose, lactitol, lactose, methylcellulose polymers, hydroxyethylcellulose, hydroxypropylcellulose, L-hydroxypropylcellulose, hydroxypropyl methylcellulose (HPMC), sodium carboxymethyl cellulose, carboxymethylene, carboxymethyl hydroxyethyl cellulose and other cellulose derivatives, starch or modified starch, magnesium carbonate, magnesium oxide, maltitol, maltodextrins, maltose, sorbitol, sucrose, sugar, xylitol, erythritol and is not limited thereto. Preferably, it is mannitol.

In the present invention, acesulfame, sodium saccharin, aspartame, sucralose, lemon flavor, natural and/or artificial sweeteners can be used as sweetener.

Lubricant is effective to prevent agglomeration of ingredients of pharmaceutical composition and avoid sticking in tablet punches/capsule filing machine. It also increase the flowability of pharmaceutical composition. The lubricant in the present invention refers to stearic acid, stearic acid salts such as magnesium stearate, palmitic acid salts such as magnesium palmitate, oleic acid salts such as magnesium oleate, sodium lauryl sulfate, magnesium lauryl sulfate, hydrogenated vegetable oil, hydrogenated castor oil, talc, sodium stearyl fumarate, macrogols, leucine, polyethylene glycol, benzoic acid, PEG derivatives and/or mixtures thereof and is not limited thereto. Preferably it is PEG 8000.

Binder in the present invention refers to polyvinylpyrolidone (PVP, povidone), polyethylene glycols (PEG), cross linked polivinylpyrolidone, cellulose derivatives (hydroxymethyl cellulose, hydroxypropyl cellulose, carboxymethylcellulose sodium, ethyl cellulose, hidroksietil cellulose, hidroksipropil methyl cellulose), sucrose, alginic acid or sodium alginate, carbomer, cotton oil, dextrin, dextrose, guar gum, hydrogenated vegetable oil type I, magnesium aluminum silica, maltodextrin, maltose, polydextrose, polyethylene oxide, stearic acid and/or mixtures thereof and is not limited thereto.

In one embodiment of the invention, effervescent pharmaceutical composition can be in the form of powder, granule or tablet and it is preferably effervescent tablet form.

In one embodiment of the invention, pharmaceutical composition comprises Erdosteine in the range of 1% and 20%, acidic component in the range of 5 % and 50 %, basic component in the the range of 10% and 60%, filler in the range of 10% and 80 % and lubricant in the range of 0 % and 10 % by weight of total weight of the pharmaceutical composition.

In a preferred embodiment of the invention, pharmaceutical composition comprises Erdosteine in the range of 5% and 10%, acidic component in the range of 30 % and 35 %, basic component in the the range of 30% and 35%, filler in the range of 20% and 25 %, sweetener in the range of 0% and 1% and lubricant in the range of 2 % and 3 % by weight of total weight of the pharmaceutical composition.

Wet granulation method and single pot technology are utilized in the production of effervescent pharmaceutical composition comprising Erdosteine.

The present invention is further defined by reference to the following examples. The examples are not intended to limit the scope of the present invention, they should be considered in the light of the description detailed above.

### Example:

| **Ingredients** | **mg / Tablet** | **(%)** |
|---|---|---|
| Erdosteine | 300.00 | 8.00 |
| Citric Acid Anhydrate | 750.00 | 20.00 |
| Sodium Hydrogen Carbonate | 1000.00 | 26.66 |
| Sodium Carbonate Anhydrate | 120.00 | 3.20 |
| Citric Acid Monohydrate | 570.00 | 15.20 |
| Potassium Carbonate Micronized | 100.00 | 2.67 |
| Mannitol DC | 753.00 | 20.08 |
| Sucralose | 27.50 | 0.73 |
| Lemon Flavor | 30.00 | 0.80 |
| PEG 8000 | 100.00 | 2.67 |
| **Total** | **3750.50** | **100.00** |

Solid- solid dispersion was carried out with the active substance, Erdosteine, and the filler mannitol. The other excipients were then sieved through a 0.63 mm sieve and mixed by geometric dilution method. PEG 8000 was added to the mixture and mixed for 5 minutes, and tablets were compressed. The disintegrating time of the tablets is approximately 2 min and pH is 5.6. Adherence to the container surface or particles on the surface of the water are not observed. Disintegration of the active substance is achieved at 100 %. Organoleptic controls complies.

## Claims

1. An effervescent pharmaceutical composition wherein it comprises
• Erdosteine and/or pharmaceutically acceptable salts, esters, polymorphs, solvates, enantiomers and/or derivatives thereof,
• at least one effervescent acid-base couple,
• at least one pharmaceutically acceptable excipient.

2. An effervescent pharmaceutical composition according to Claim 1, wherein the acidic component of effervescent acid - base couple is selected from, citric acid, tartaric acid, absorbic acid, fumaric acid, malic acid, adipic acid and/or mixtures thereof.

3. An effervescent pharmaceutical composition according to Claim 1 or Claim 2, wherein the acidic component of effervescent of acid-base couple comprises citric acid anhydride and citric acid monohydride.

4. An effervescent pharmaceutical composition according to Claim 1 or Claim 2 or Claim 3, wherein effervescent pharmaceutical composition comprises acidic component in the range of 15% and 55% by weight of the total weight of the composition.

5. An effervescent pharmaceutical composition according to Claim 1 or Claim 2 or Claim 3 or Claim 4, wherein effervescent pharmaceutical composition comprises acidic component at 35 % of the total weight of the composition.

6. An effervescent pharmaceutical composition according to Claim 1, wherein the basic component of effervescent acid-base couple is selected from sodium bicarbonate, sodium carbonate, potassium bicarbonate, potassium carbonate, sesquicarbonate, amorph calcium carbonate, calcium carbonate and/or mixtures thereof.

7. An effervescent pharmaceutical composition according to Claim 1 or Claim 6, wherein effervescent pharmaceutical composition comprises basic component in the range of 10% and 60% by weight of the total weight of the composition.

8. An effervescent pharmaceutical composition according to Claim 1 or Claim 6 or Claim 7, wherein effervescent pharmaceutical composition comprises basic component at % 32 by weight of the total weight of the composition.

9. An effervescent pharmaceutical composition according to Claim 1, wherein the effervescent pharmaceutical composition is administered orally.

10. An effervescent pharmaceutical composition according to Claim 1, wherein effervescent pharmaceutical composition is in the form of effervescent tablet.

11. An effervescent pharmaceutical composition according to Claim 1, wherein it comprises filler, lubricant, sweetener and/or binder as excipient.

12. An effervescent pharmaceutical composition according to Claim 1 or Claim 11, wherein the filler is selected from calcium carbonate, calcium phosphate, dibasic calcium phosphate, tribasic calcium sulfate, calcium carboxymethyl cellulose, cellulose, dextrin derivatives, dextrin, dextrose, fructose, lactitol, lactose, methylcellulose polymers, hydroxyethylcellulose, hydroxypropylcellulose, L-hydroxypropylcellulose, hydroxypropyl methylcellulose (HPMC), sodium carboxymethyl cellulose, carboxymethylene, carboxymethyl hydroxyethyl cellulose and other cellulose derivatives, starch or modified starch, magnesium carbonate, magnesium oxide, maltitol, maltodextrins, maltose, sorbitol, sucrose, sugar, xylitol, erythritol.

13. An effervescent pharmaceutical composition according to Claim 1 or Claim 11 or Claim 12, wherein filler is mannitol.

14. An effervescent pharmaceutical composition according to Claim 1 or Claim 11, wherein lubricant is selected from stearic acid, stearic acid salts such as magnesium stearate, palmitic acid salts such as magnesium palmitate, oleic acid salts such as magnesium oleate, sodium lauryl sulfate, magnesium lauryl sulfate, hydrogenated vegetable oil, hydrogenated castor oil, talc, sodium stearyl fumarate, macrogols, leucine, polyethylene glycol, benzoic acid, PEG derivatives and/or mixtures thereof.

15. An effervescent pharmaceutical composition according to Claim 1 or Claim 11 or Claim 14, wherein lubricant is PEG derivatives.

16. An effervescent pharmaceutical composition according to Claim 1 or Claim 11, wherein the sweetener is selected from acesulfame potassium, sodium saccharine, aspartame, sucralose, xylitol, lemon flavor and natural and/or artificial sweeteners.

17. Effervescent pharmaceutical composition according to Claim 1 or Claim 11, wherein binder is selected from polyvinylpyrolidone (PVP, povidone), polyethylene glycols (PEG), cross linked polivinylpyrolidone, cellulose derivatives (hydroxymethyl cellulose, hydroxypropyl cellulose, carboxymethylcellulose sodium, ethyl cellulose, hidroksietil cellulose, hidroksipropil methyl cellulose), sucrose, alginic acid or sodium alginate, carbomer, cotton oil, dextrin, dextrose, guar gum, hydrogenated vegetable oil type I, magnesium aluminum silica, maltodextrin, maltose, polydextrose, polyethylene oxide, stearic acid and/or mixtures thereof.
